# NEW EUROPEAN PATENT SPECIFICATION

(11) **EP 0 346 116 B2**
(45) Date of publication and mention of the opposition decision: **02.01.1997**
(45) Mention of the grant of the patent: 07.09.1994
(21) Application number: 89305788.5
(22) Date of filing: 08.06.1989
(51) Int. Cl.: A61F 9/00

(54) **Apparatus for laser sculpture of the cornea**
Vorrichtung zur Laserformung der Hornhaut
Dispositif pour modifier la surface cornéenne par laser

(30) Priority: 09.06.1988 US 204504; 23.02.1989 US 314654
(43) Date of publication of application: 13.12.1989
(73) Proprietor: VISX INCORPORATED, Santa Clara, CA 95051-0703 (US)
(72) Inventor: Yoder, Paul R., Jr., Wilton Connecticut 06897 (US)
(74) Representative: Milhench, Howard Leslie

(56) References cited:
- EP-A- 0 207 648
- EP-A- 0 218 427
- EP-A- 0 224 322
- EP-A- 0 257 836
- EP-A- 0 274 205
- DE-A- 2 747 618
- DE-A- 3 414 986

## Description

### BACKGROUND OF THE INVENTION

The invention relates to that aspect of ophthalmic surgery which is concerned with operations upon the external surface of the cornea.

EP-A-0207648 and EP-A-0257836 describe methods and apparatus for selective and progressive laser ablation of corneal tissue whereby to sculpt a new optically corrected curvature in the optically used portion of the anterior surface of the cornea. Specific illustrations are given for effecting such optically correcting change, in the cases of myopia, hyperopia and astigmatism. The involved principles are applicable for any ablation-producing wavelength, but the specific illustrative disclosure is for use of ultraviolet radiation, in the range of 200 nanometers or less. Reference is made to said patents and to the patent applications referred to therein, for a more complete discussion of tissue ablation and of various techniques for effecting desired corneal-curvature changes.

It sufficies for present purposes to explain that the techniques disclosed in said patents may be generically described as employing varying laser-spot size at impingement with the cornea, in the course of a single surgical procedure. According to one mode, a zoom lens is the means of progressive variation of spot size; if the spot is circular and of progressively varying radius, ablation is greater on the optical axis of the eye, and ablation reduces with increasing radius, thus providing a myopia-correcting curvature change in the anterior surface of the cornea. According to another mode, an indexible mask has a progressive development of apertures of varying size which, for the case of myopia correction, are circular and of progressively varying radius.

Each of these modes is subject to at least one limitation which is believed to prevent realization of optimum results. In the case of zoom-lens reliance for spot-size variation, the desired range of spot-size variation can be as much as 5:1 or 10:1, but the greater the magnification range of a zoom-lens system, the greater the range of laser-beam flux density deliverable to the cornea, i.e., flux-density reduction is a function of increasing magnification. In the case of an indexed-aperture mask, the ablated ultimate new curvature is in reality a series of stepped penetrations which only approximate a smooth curvature.

What has been said, as to use of said patents for tissue-ablating laser surgery to reduce myopia, applies also (1) for varying annular-spot projections to reduce hyperopia and (2) for varying slit-width projections to reduce astigmatism.

### BRIEF STATEMENT OF THE INVENTION

It is an object of the invention to provide an improved apparatus for use in surgically operating upon the outer surface of the cornea, the apparatus of the invention enabling a materially smoother reprofiling of the optically used region of the cornea to be achieved.

According to the present invention there is provided apparatus for performing ophthalmological surgery by selective ablation of the optically used region of the anterior surface of the cornea with penetration into the stroma to achieve a volumetric removal of corneal tissue providing for corneal curvature correction, said apparatus comprising laser means for producing an output beam having an intensity which is limited per unit time to ablate but a fraction of a predetermined maximum ablation depth into the stroma, means for directing said laser output beam onto the eye which is to be treated, and control means for adjustably determining the laser beam cross-section at corneal impingement in said optically used region in a controlled manner in the course of a surgical treatment so that sculpturing action for curvature correction results from a controlled variation in the area of the cornea that is irradiated and a corresponding controlled variation in the cumulative ablative flux delivered to different areas of said optically used region of the cornea so as to obtain said corneal curvature correction, as known from EP-A-0207648 abovementioned, the apparatus according to the invention being characterized in that said control means comprises the combination in the laser beam path of at least one mask having a plurality of mask apertures of different areas that are selectively introducible into the laser beam path for correspondingly determining the laser beam cross-section at corneal impingement and a variable magnification zoom lens, and means arranged to controllably adjust the setting of the zoom lens in the course of corneal irradiation through each mask aperture in a series of progressively different area mask apertures in order to further subdivide the steps of progression of the laser beam cross-section at corneal impingement that are achieved by irradiation of the optically used region of the cornea through said series of different mask apertures thereby to obtain a substantially smooth change in corneal curvature profile.

As is explained in the following, the invention achieves the above-stated object by combining the varying spot-size capabilities of a variable magnification (zoom) lens with those of an indexible mask having a progression of variously sized apertures, designed as appropriate for the particular curvature-reducing or curvature-increasing sculpture that is prescribed for a given patient's optical-curvature problem. The zoom-lens settings are varied in the course of cornea exposure through each of the succession of mask apertures. To do this, the range of zoom-lens magnification can always be less than 2:1, yet the zoom-lens system can effectively reduce to insignificance the stepped profiling that is inherent in reliance only upon the indexed aperture technique. And, for maintenance of substantially constant delivered laser flux density, the output beam of the laser can be attenuated in inverse relation to the instantaneous magnification of the zoom system.

Still further, for situations in which a Gaussian profile characterizes the effective distribution of flux density across the operative section of the ablating laser beam, the invention provides for such cooperative use of a suitable indexible mask, zoom-lens system and laser-beam attenuator as to produce not only desired optical-curvature corrections but also sculpture in depth to reproduce original curvature, as for the acceptance of a corneal transplant, or for the removal of a scar or other anterior-surface damage to the cornea.

EP-A-0207648, as aforementioned, does disclose the use of a zoom lens to achieve spot size variation in an ophthalmological laser surgery apparatus and, in an alternative embodiment, discloses the use of an indexible mask having a progression of differently sized apertures. However, EP-A-0207648 does not disclose the combination of a zoom lens with one or more indexible masks and did not foresee the advantages that such a combination could provide as is explained hereinafter. EP-A-0257836 has a similar disclosure to EP-A-0207648 and additionally discloses in Fig. 31 the combination of a continuously adjustable masking device with a zoom lens. However, in the arrangement of Fig. 31 of EP-A-0257836 the continuously adjustable masking device is responsible for varying the laser beam cross-section at impingement on the optically used region of the cornea in order to achieve corneal curvature correction, and the zoom lens is responsible only for expanding the laser beam in order to sculpt a profile smoothing annulus around the operative, optically used area thereby to promote epithelial regrowth. EP-A-0257836 does not disclose the combination of a zoom lens with one or more indexible masks, did not address the problem which the present invention addressed and, as with EP-A-0207648, did not foresee the advantages that the present invention provides.

### DETAILED DESCRIPTION

The invention will be described in detail for a preferred embodiment, in conjunction with the accompanying drawings, in which:
Fig. 1 is a schematic diagram in perspective, to show the general arrangement of operative components of the invention;
Fig. 1A is a fragmentary detail of a modified arrangement for a part of Fig. 1;
Fig. 2 is an enlarged half-section profile of the anterior surface of an eye having a hyperopia problem, together with an indication of progressively stepped ablation to achieve hyperopia reduction via an indexed-aperture mask of said L'Esperance, Jr. patents;
Fig. 2A is a fragmentary plan view of an indexible aperture mask of the invention, said mask being a component of Fig. 1;
Fig. 3 is a simplified optical diagram to permit initial discussion of principles of the invention;
Fig. 4 is a more specific optical diagram in support of further discussion of principles;
Fig. 5 is a table of mask-opening diameters applicable to an illustrative use of Fig. 2;
Fig. 5A is a table of mask-opening diameters, iris settings, and zoom-lens settings for achieving the improvement of the invention, using apparatus of Figs. 1 and 2A;
Fig. 6 is a view similar to Fig. 2, to permit discussion of use of the invention to reduce a myopia condition;
Fig. 6A is a fragmentary view similar to Fig. 2A, showing an indexible-aperture mask for reducing the myopia condition of Fig. 6;
Fig. 7 is another fragmentary view of an indexible-aperture mask, similar to Figs. 2A and 6A, but specifically applicable to ablation to reduce an astigmatism condition;
Figs. 8, 8A and 8B are similar diagrams in the style of Fig. 2, to illustrate preparation for a corneal transplant; and
Fig. 9 is another diagram in the style of Fig. 2, to illustrate use of the invention to eliminate the effect of scar damage to the cornea.

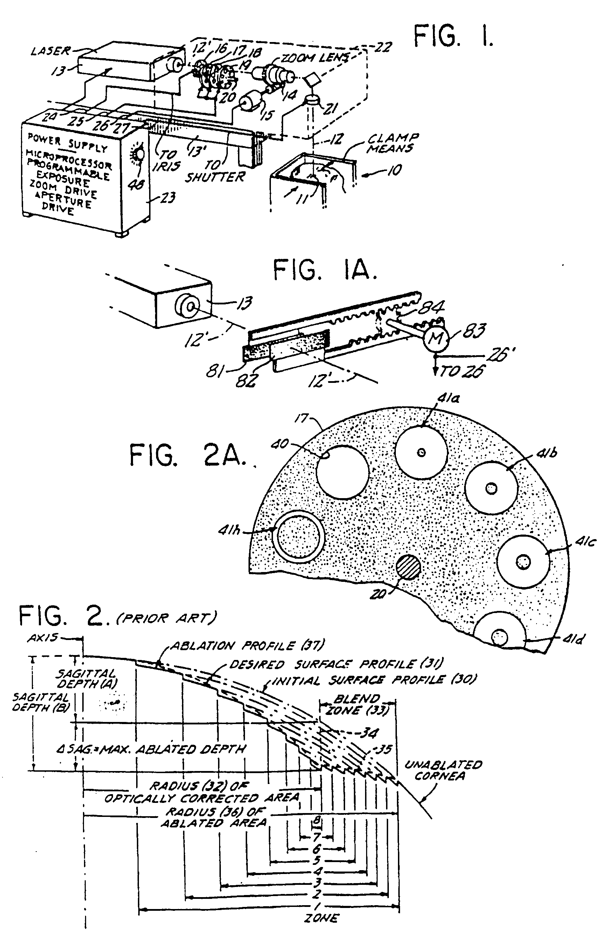

In Fig. 1, clamp means 10 is shown for fixed retention of the head of a patient (reclined, face up) such that the eye 11 to be operated upon is fixedly aligned with a downwardly folded portion 12 of the central axis 12' of beam output from a stationary laser device 13, supported by a table or other base 13'. The optical system of laser-beam projection to eye 11 includes zoom-lens means 14 having a reversible motor drive 15 whereby laser-spot size at eye 11 can be caused to vary. The optical system is further shown in Fig. 1 to include a variable-aperture iris 16, three aperture-mask discs 17, 18, 19 each of which is selectively indexible about an axis 20 offset from and parallel to beam 12', and a selectively operable shutter 21. The described optical-system components are preferably contained within a housing 22, which is only suggested by dashed lines, which will be understood as having entrance-port and exit-port windows as necessary to permit laser-beam delivery on alignment 12 to eye 11.

A cabinet 23 is shown by legend to include a power supply for the laser, and cabinet 23 is also shown by legend to include programmable microprocessor means for controlling exposure and beam (spot) size on axis 12 at impingement with the anterior surface of the cornea of eye 11, as will later become clear. Specific connections shown from cabinet 23 include line 24 for control of iris 16, a multiple-line cable 25 for selective indexing control of each of the discs 17, 18, 19, line 26 for reversible-drive control of zoom-lens magnification via motor 15, and line 27 for open/shut operation of shutter 21.

As in the abovementioned patents, clamp means 10 preferably includes means to stabilize the patient's head; this is schematically suggested by legend to be via opposed engagements at the region of his temples. An eye-retaining fixture such as shown and described in said patents may be used to peripherally engage eye 11 at the cornealscleral area.

The laser selected for use at 13 emits at a tissue-ablating wavelength, which may be in the narrow water-absorption infrared band at or near 2.9 microns, but which is preferably in the far-ultraviolet region, as provided by an argon-fluoride laser at 193 nanometers. Various gas lasers operate at tissue-ablating ultraviolet wavelengths, and frequency-multiplying techniques applied to other lasers, including crystal lasers, provide further alternative sources.

One of the existing commercial excimer-laser products of Lambda Physik GmbH, Göttingen, Federal Republic of Germany, for example, their Model EMG 103 operating with argon-fluoride, is satisfactory for use as laser 13. Also satisfactory is one of the Series-2000 excimer-laser products of Questek, Inc., Billerica, Massachusetts, operating with argon fluoride. It will be understood that the output beam of such lasers is rectangular and that such an output beam may be implemented by beam-expansion and homogenizing techniques, such that, for present purposes, the flux-density of laser-beam output will have been uniformly distributed across a square or circular beam section, prior to delivery on axis 12' to the spot-size control means within housing 22. It will also be understood that optical elements of lens 14 and other components transparent to involved laser radiation are of suitable materials, such as fused silica, calcium fluoride, and magnesium fluoride. Techniques of beam shaping and homogenization need not now be described, but are disclosed in European Patent Application No. 88300789 (EP-A-0280414).

Fig. 2 has been labelled "Prior Art" because it is virtually entirely devoted to showing the hyperopia-correcting operation of an indexible aperture-disc embodiment of said EP-A-0207648, and because this capability of said patent is the context from which to explain the present invention. In the Fig. 2 situation, the hyperopic eye has an initial anterior-surface profile 30 of too-great radius of spherical curvature, and it is desired to reduce the radius of spherical curvature into substantial conformance with a desired-surface profile, shown by dashed line 31, to the limiting extent of prescribed radius 32 of the optically used area of the cornea. As can be seen from legends in Fig. 2, such a desired optical correction proceeds from an initial sagittal depth A to a maximum sagittal depth B, involving an increase (Δ SAG.) of maximum ablated depth at the outer radius 32 of the optically corrected area; and were it not for providing a blend zone 33 of ablation radially outside and contiguous to the optically corrected area, a sharp annular wall 34 would be left to delay or frustrate post-surgical regrowth of epithelium over the ablated area. The desired blend-zone profile is indicated by dashed line 35, providing a gently sloped profile of transition out to the outer unablated region of the cornea.

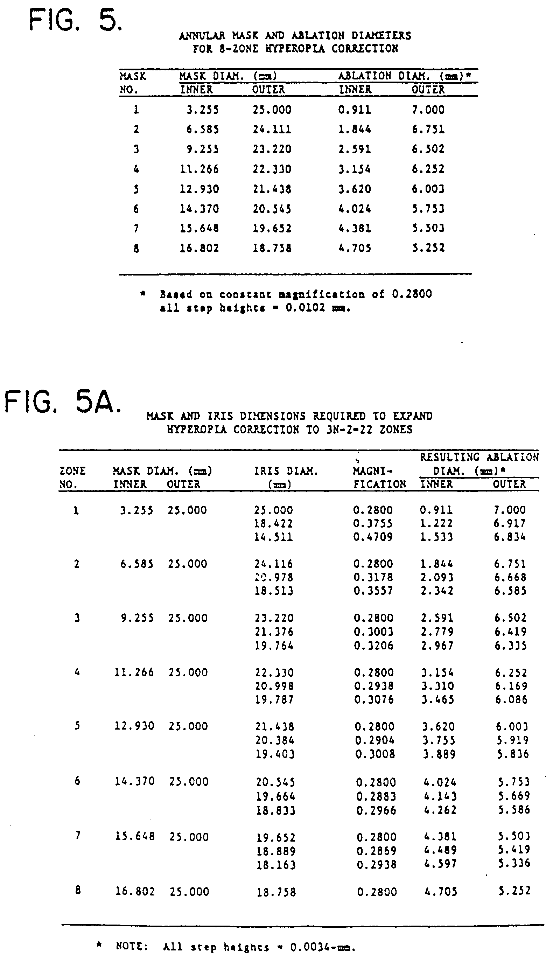

In the example of Fig. 2, the indexible aperture disc provides eight annular apertures, and the exposure time (number of excimer-laser pulses) of laser radiation delivered to the cornea is the same for exposure through each of the successive annular apertures. The inner and outer radii, i.e., the annular zone, for each of these annular apertures, are shown by legend; and as explained in EP-A-0207648, each annular aperture is an opaque central spot, concentric within an outer circular limit or transparency of a suitable substrate which has been coated for opacity outside said limit. Experience has confirmed that, for argon-fluoride excimer lasers, as specified above, and as delivered, at appropriate and essentially constant energy density, through beam-homogenizing and shaping optical elements of the system described in EP-A-0280414, each excimer-laser pulse will ablate approximately 0.1-micron of incremental depth penetration into corneal tissue; thus, 100 such pulses at a given indexed aperture position will ablate an annulus of approximately 0.01-mm penetration depth. Fig. 5 tabulates descriptive data on aperture size and ablation diameters for the eight-zone indexible-disc station depicted in Fig. 2, wherein there is only one optical system, of magnification 0.2800, interposed between the indexible disc and the cornea, and individual steps of the resulting ablation profile 37 are of 0.0102-mm height. It is to be cautioned that the tabulated figures assume uniform ablation depth of all steps of Fig. 2. However, theory and experience indicate that ablation depth effectively reduces in accordance with a cosine function of angle of laser-beam incidence on the cornea, reducing roughly 10 percent at radius 32, as compared with full-step height for the inner radius of zone 1. This effect can be compensated by appropriate small increases in number of pulses for the larger outside diameter apertures.

In accordance with the invention, step height (which is a function of (1) the number of successive mask apertures of the prior art, (2) the number of laser pulses per mask opening, and (3) the energy density of the laser beam at the cornea) is reducible to the extent of at least an order of magnitude when an apertured mask is used in conjunction with a zoom lens, as at 14, the latter being used solely as a means of effectively finely subdividing the steps of progression of beam-spot size at the cornea, between successive apertures of a given aperture disc 17, 18 or 19, each of which is of specialized configuration to serve the respective purposes of hyperopia reduction, myopia reduction, and astigmatism reduction. Since only one of the discs 17, 18, 19 is to be used for a given operation, each of these discs has one large and fully open indexible-aperture location, as at 40 in the case of hyperopia-reducing disc 17 of Fig. 2A, which location is indexible into concentric relation with beam axis 12' to permit use of a selected one of the two other discs 18, 19.

Also, in accordance with a feature of the invention and here specifically illustrated for the case of blend-zone (33) formation along with a hyperopia-reducing ablation procedure, an iris, as at 16, is variably controlled to define the progression of outer-diameter limitation delineated for the respective zones of the successive indexible apertures. This iris function at 16 will be understood to be smoothly continuously variable or in stepped progression, under microprocessor control and in synchronism with coordinating microprocessor control of disc (17) indexing and of zoom-lens setting, the latter being continuously variable or in stepped progression within a relatively narrow range of magnification change between any two adjacent indexible apertures of disc 17.

The programmed use of iris 16 to determine the succession of outer-diameter limits of annular-spot impingement at the eye necessarily means that the outer-diameter limits of the succession of annular apertures 41a, 41b, 41c... 44h are not controlling of delivered spot size; they are therefore seen in Fig. 2A to have the same maximum outer diameter which may be equal to the outer diameter of the fully open aperture 40. This condition is further supported by the illustrative tabulation of Fig. 5A, wherein iris-diameter settings and zoom-lens magnification settings are set forth in the context of constant maximum outer-diameter dimensioning of all eight zone apertures of disc 17.

It has been observed that both iris 16 and zoom-lens 14 are capable of continuous variation, and it will be understood that microprocessor means at 23 may be programmed to achieve such continuous variations, in suitably correlated synchronism with each other, for each of the zone-identifying indexible apertures of disc 17. However, the principle of step-size reduction through coordinated use of iris 16 and zoom lens 14 is adequately illustrated by Fig. 5A, for purposes of comparison with the prior-art technique of Fig. 5, by using only three zoom and iris settings for each of the eight zone-indexed positions in a full 8-zone succession of using disc 17 (Fig. 2A). Thus, in Fig. 5, the first iris setting and the first zoom-lens setting, for each newly indexed zone-identifying aperture of disc 17, will be seen to be respectively equal to the corresponding outer disc-aperture diameter of Fig. 5 and to the constant magnification (0.2800) of Fig. 5. And between adjacent indexed-aperture openings, two stages of small incremental iris-opening changes and relatively small incremental magnification changes account for a trebling of the number of different spot sizes with which to accomplish the hyperopia-reducing ablation process. With a trebling of the number of different spot sizes, one accomplishes step-size reduction by a factor of three. And it should be clear without further explanation that to provide, say ten steps of iris setting and of coordinated zoom-lens setting for each indexed-aperture position of disc, will provide a 10-fold increase in the number of discrete steps per procedure, as well as a 10-fold reduction in the size of individual steps, thus reducing to negligible proportions any deviation in ultimate desired profile with respect to the dashed-line profiles 31, 35 of Fig. 2.

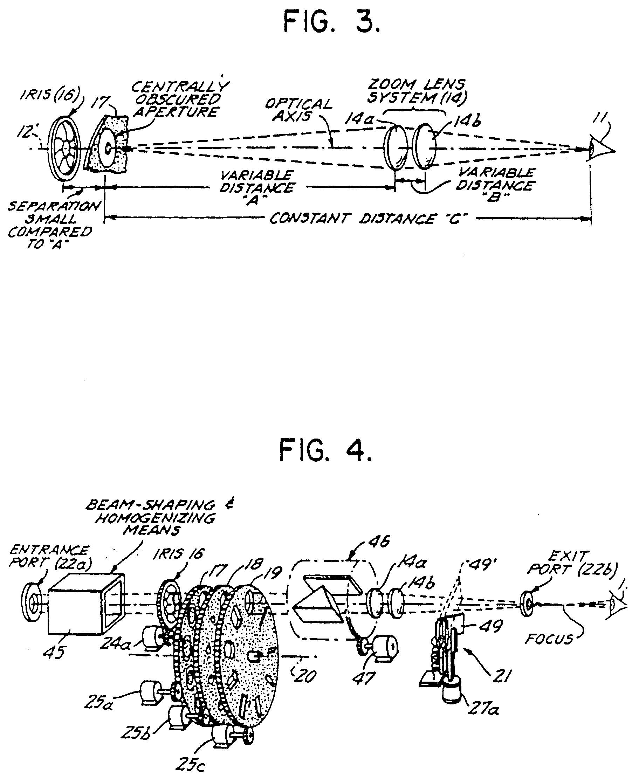

Fig. 3 serves to indicate basic relationships between optical components which determine spot-size control in the hyperopia-correction situation wherein one of the apertures 41a... 41h of disc 17 has been indexed into concentric relation with the central axis 12' of laser-beam projection. The zoom-lens system 14 operates over the constant object/image distance C and is seen to comprise two optical elements (or groups of optical elements) 14a, 14b which image, at eye 11, the central opaque area of the currently operative indexed annular aperture of disc 17; the imaging process involves an image reversal, which is of no adverse consequence for present purposes. Changes in image magnification result from motor (15) driven variation of the separating distance B between elements 14a, 14b as well as synchronized variation of the separating distance A between the aperture 17 and the lens 14a. And since iris setting is determinative of the currently effective outer diameter of the currently indexed aperture of disc 17, it is indicated by legend that axial separation between iris 16 and disc 17 is small compared to the variable object distance A, thus dictating a preference that the hyperopia-reduction disc 17 be adjacent to iris 16, and that remaining discs 18, 19 be at similarly small axial spacing from each other and from disc 17.

Fig. 4 provides further detail of the means for spot-size control, between the laser-beam entrance port 22a of housing 22 and the exit port 22b of beam projection to eye 11, it being understood that the beam-folding mirror of Fig. 1 has been omitted for simplifying purposes. Thus, within housing 22, beam-shaping and homogenizing means 45 will be understood preferably to deliver to iris 16 a virtually collimated laser beam of circular section which is characterized by substantially homogeneous distribution of flux density, the circular section being large enough to circumferentially continuously lap iris 16 for its largest diameter opening, so that this largest iris opening can thereafter be determinative of the maximum outer diameter of aperture opening in any and all of discs 17, 18, 19. Iris (16) setting is shown to be variably operated by a reversible drive motor 24a which will be understood to be served by line 24 of Fig. 1. Each of the three indexible aperture discs 17, 18, 19 is shown to be independently positionable by its own indexing-drive motor 25a, 25b, 25c, respectively served by a different control-circuit line in cable 25 of Fig. 1. An image-rotating optical system 46 is bodily rotatable, as via edge-driving coupling to a drive motor 47 which will be understood to be controllable by knob setting 48 (at 23 in Fig. 1) for an astigmatism-correcting procedure to be described. The zoom-lens elements 14a, 14b and their relationships A, B, C have already been described in connection with Fig. 3. And, finally, shutter 21 is seen to comprise a pivoted opaque blade 49 which is spring-urged to closed position (49') and which is actuated by solenoid means 27a to open position, under microprocessor control via line 27 of Fig. 1.

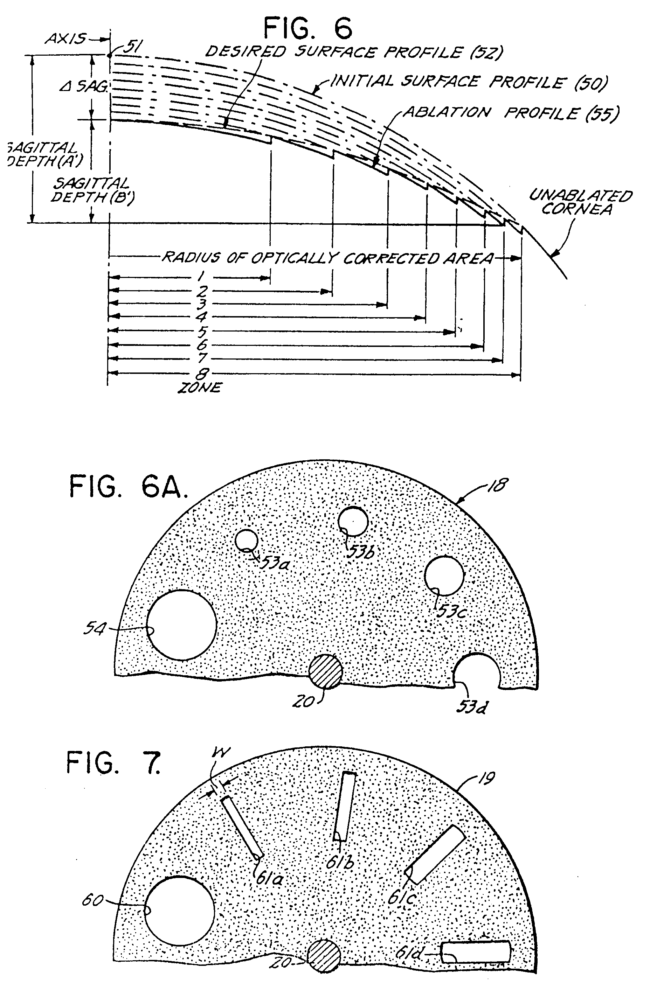

Figs. 6 and 6A illustrate myopia-reducing use of the invention with simplified drawings that are analogous to Figs. 2 and 2A. The myopia-inducing initial anterior-surface profile 50 is, at intercept 51 with the optical axis of the eye, taken as the reference point for defining the sagittal depth A' of the optically used area of the eye (per profile 50) which is to be correctively ablated to a desired new profile 52 of greater spherical radius of curvature, with reduced sagittal depth B'. And maximum ablation depth (Δ SAG.) is at the central region of the eye. The first eight of nine apertures 53a, 53b, etc. of indexible disc 18 are circular and of progressively increasing diameter, consistent with the progression of zone radii shown in Fig. 6; the ninth aperture 54 is at least as large as the corresponding large opening 40 in disc 17, thus permitting myopia-correcting functions to be suppressed while one of the other discs 17, 19 is determinative of the selected sculpturing procedure. Without the coordinated zoom-lens functioning described for use in connection with Fig. 2A, the successively indexed myopia-reducing operation of disc 18 will sculpt a succession of eight stepped increments of tissue ablation, which could result in steps as large as 0.01-mm high in the resulting ablation profile 55, for the illustrative exposure time (number of pulses) assumed in the discussion of hyperopia correction. But with zoom-lens setting so controlled by the microprocessor as to substantially enlarge (e.g., by a factor of ten) the number of progressively changed step areas for each indexed one of the openings 53a, 53b, etc., the step-height increments become literally insignificant, and the desired-surface profile 52 is closely approached if not effectively realized.

Fig. 7 is a view similar to Fig. 6A to illustrate a third indexible aperture disc 19, which is dedicated to reduction of astigmatism. As with the other discs, there are plural equally spaced apertures, one of which is a large-area circular opening 60, to permit operative use of one of the other two indexible-aperture discs 17, 18. For astigmatism reduction, each of the succession of openings 61a, 61b, 61c, etc. is a radially extending slit which is indexible into operative position, centered on the axis 12'; slit openings 61a, 61b, etc. are of length which may correspond with the diameter of large opening 60, and of lateral width W which progressively increases, for each successively indexed position of disc 19. Thus, if the sagittal depth of astigmatism-reducing ablation (Δ SAG.) is to be equivalent to that shown in Fig. 6 for myopia-reducing ablation, Fig. 6 may be taken as a half-section profile of the cylindrical curvature to be reduced for astigmatism correction. The cumulative effect of a full cycle of indexed disc-19 use will again be a stepped cylindrical ablation profile (as at 55), with eight relatively high steps, and at a certain angular orientation when imaged at the eye. The angle of orienting the beam rotator 46 of Fig. 4 will determine ultimate orientation of cylindrical ablation at the eye, and a prescription orientation of cylindrical-axis orientation may be selected by adjustment of knob 45 at control console 23 of Fig. 1. Again, it will be appreciated that by suitably programmed zoom-lens magnification control for each of the eight indexible slit openings of disc 19, a very much improved resultant profile of reduced cylindrical curvature is achieved, illustratively with an order of magnitude approach to the desired profile.

In all of the foregoing discussion of iris, zoom-lens, and indexible-disc control to achieve a selected one of the three illustrative curvature changes, it has been assumed, for simplification purposes, that equal steps (i.e., abated penetration depth increments) are to be achieved for each of the respective indexed positions of the selected indexible disc. As seen in Figs. 2 and 6, this means that radial increments for each of the steps near the optical axis of the eye are larger than for each of the steps near the outer radius of the optically corrected area. Clearly, these radial increments are subject to choice, to the extent that, if desired, they may all be equal, or they may be smaller near the optical axis and larger near the outer radius of the optically corrected area, and the magnification control of zoom-lens setting may be selected as appropriate to achieve virtually any fineness of subdividing step allocation for each of the indexed-aperture positions of the involved one of discs 17, 18, 19.

A typical cycle of laser-beam exposure to achieve a given curvature correction, all under microprocessor control, can be as follows:
(1) With shutter closed, with a particular disc 17, 18, or 19 selected for its particular sculpturing function, and with the number of exposure pulses selected to achieve the maximum tissue-ablating depth for the prescribed diopter change at a given maximum radius of optically corrected area, initiate laser operation and check its output for specified amplitude and homogeneity of flux distribution. As a result of such selection, the two remaining discs (which are not to be used) will have been automatically indexed to place their large-opening positions concentric with the beam axis 12', and the selected disc will have been indexed to place the initial one of its eight progressively varying apertures on the beam axis.
(2) Operation is initiated by opening shutter 21, for a period of time (number of pulses) which reflects measured exposure through the first of the eight progressive apertures, while zoom-lens settings (and, if disc 17 is used, also iris-aperture settings) are modified pursuant to microprocessor control. At microprocessor-determined conclusion of exposure for the first indexible aperture position, shutter 21 is closed, and under microprocessor control, the selected disc is advanced to the next indexed aperture, while zoom-lens setting (and, if applicable, iris-aperture setting) is re-established as needed for use at the second index position of the selected disc, whereupon shutter 21 is automatically opened to permit exposure for the second phase of controlled variation of zoom magnification (and, if applicable, iris-aperture variation).
(3) Cycles as in (2) above are program-controlled to repeat, as appropriate for each of the eight indexible positions of the selected disc, each being accompanied by controlled variation zoom-magnification (and, if applicable, iris-aperture variation).
(4) Laser 13 is automatically shut down at shutter 21 closure, upon completion of the requisite number of cycles, being eight for the illustrative case of eight indexible-mask apertures for each disc.

As explained above, the zoom-lens/indexed-aperture example of Fig. 5A represents simplification, in that the same exposure time (number of pulses) is used for each indexed setting of the indexible-mask wheel (17, 18 or 19) This necessarily means that for each indexed-aperture position, the zoom-lens magnification proceeds through the greatest range of variation for aperture 41a, as compared with subsequently indexed apertures.

As another means of distributing laser-beam exposure to the cornea for a given procedure of optical curvature change, e.g., for more equal increments of radius of ablation throughout the entire optically corrected area to be ablated, it is possible to so select the number of mask apertures and the increments of mask-aperture dimensions that, in conjunction with continuously varying zoom operation and with different numbers of pulses allocated to each indexed aperture, more nearly uniform increments of ablated radius are achieved throughout the full course of the procedure. For example, in a myopia-correcting situation, four different indexed apertures (53a, 53b, 53c, 53d) can suffice if selected at four successive radius-doubling increments of radius. Specifically, four such apertures, illustratively at 1.562-mm, 3.125-mm, 6.250-mm, and 12.500-mm diameter, can be indexed-programmed for pulsed utilization with zoom lens 14 to require no more than a 1:2 zoom-lens range of continuous magnification change, for expanding-magnification action at each indexed aperture, so as to achieve a combined range of zoom-lens and indexed-disc coaction, from 1.562-mm diameter to 25.000-mm diameter, corresponding to 0.437-mm to 5.000-mm ablation diameter, where the 1:2 range involves limiting magnifications of 0.200 and 0.400, respectively. For this range, and for an approximately 3-diopter reduction in myopia, laser-pulse allocation may illustratively be:
-- 20 pulses for the 1.562-mm aperture with zoom control of the ablation range 0.312-mm to 0.625-mm diameter.
-- 40 pulses for the 3.125-mm aperture, with zoom control of the ablation range 0.625-mm to 1.250-mm diameter.
-- 80 pulses for the 6.250-mm aperture, with zoom control of the ablation range 1.250-mm to 2.500-mm diameter.
-- 160 pulses for the 12.500-mm aperture, with zoom control of the ablation range 2.500-mm diameter to 5.000-mm diameter.
It will be seen that in this illustrative case, the order of magnitude of incremental radial expansion, per pulse, for a 300-pulse treatment, is 0.015-mm per laser pulse.

It is to be understood that all examples given in the foregoing discussion are for illustrative purposes only, to show inter alia that the equal-step concept of the prior art, as per profile 37 of Fig. 2 or as per profile 55 of Fig. 6 is not to be considered mandatory or even preferred, in the programmed coordination of zoom-lens control, with indexing control and with indexed-aperture size selection. The selection of coordinated control has been shown to be also achievable on the basis of substantially uniform incrementing of ablating radius throughout a given surgical procedure, without imposing more than a 1:2 magnification-range capability on the zoom lens. Other types of programs are also devisable, the important point being that an almost perfectly smooth new curvature profile is made possible in spite of changing flux density, by relying upon a predetermined program of indexing the mask apertures of a given disc (17, 18 or 19) in coordination with laser-exposure time (i.e., number of pulses) at each indexed position and in coordination with the particular zoom-magnification range desired for each indexed-aperture position. It will be further understood, that, depending upon the program selected for a given hyperopia-reducing procedure, the aperture control of iris 16 must also be coordinated with zoom-lens magnification, to achieve the smooth blend profile 35, for the larger radius outside the radius 32 of the optically corrected area.

Also, by way of example, with magnification ratios always within the range 1:1.414, and providing stepped aperture increments of radius accordingly, the range of density variation with a given zoom use of any single aperture is maintained within the range 1:2.

The foregoing discussion involves different examples of sculpturing procedures which employ indexed apertures in conjunction with controlled variation of zoom-lens magnification, and is also illustrative of how parameters of a given program can be selected to minimize the range within which magnification varies, in the course of a given procedure. This is an important factor in the controlled ablation of corneal tissue, using the irradiating output of an excimer laser of the character indicated above, wherein laser-beam flux density should be held within relatively narrow limits, such as a dynamic range of 4:1 if ablation is to be reliably achieved. Preferably, the flux density of the beam at impact with corneal tissue should be as near constant as is possible.

To achieve substantially constant flux density at the cornea, in the circumstance of varying magnification, a synchronizing provision can be built into the microprocessor program whereby laser 13 is controlled for increasing output intensity as a synchronized function of increasing zoom-lens magnification, using control connections schematically shown in Fig. 1, between the power-supply/microprocessor unit 23 and the respective laser (13) and zoom-lens adjustment (15) means. Alternatively, a single optical filter with rotational or uniaxial spatially variable optical density could be interposed between the point of laser (13) output and the spot-size determining elements (16, 17, 18, 19), or in any event positioned upstream from the zoom lens 14, and such optical filter can be displaceably driven to variably attenuate the laser beam in inverse relation to the instantaneous magnification of the zoom lens 14. However, fragmentary diagram of Fig. 1A, shows a present preference for an arrangement of two such filters 81, 82 acting in opposition upon axis 12' and driven by a motor 83 having suitable synchronizing connection 26' to the connection 26 for zoom-lens control. Specifically, shading for filter 81 is indicative of reducing density from left to right, and shading for an identical filter 82 is indicative of increasing density from left to right; and a reversible motor 83 for differential rectilinear rack drives, via pinion (84) engagement to both racks, assures balance in attenuation across the beam aperture of laser 13.

With the synchronized attenuation function achievable via the adjustment at 81, 82, 83, the laser 13 need only be set for a slightly greater output intensity than needed for ablation purposes, because attenuation functions of filters 81, 82 and of zoom-lens magnification will be in such synchronized inverse relation as to assure desired substantially constant delivery of tissue-ablating flux-density to the cornea at all operational phases of a given procedure.

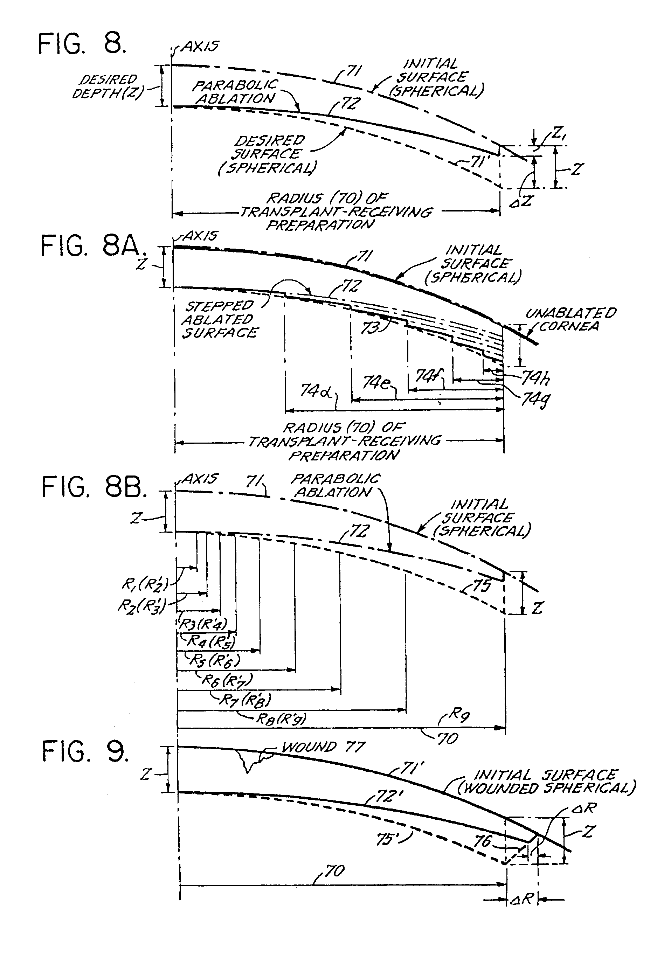

Figs. 8, 8A and 8B are similar half-section profile diagrams as in Fig. 2 but in illustration of utility of the invention for purposes of preparing a cornea for reception of a corneal transplant, in the circumstance wherein the laser beam is not characterized by sufficiently homogeneous flux (intensity) distribution. EP-A-0207648 and EP-A-0257836 describe preparation for a corneal transplant, i.e., a straight constant-diameter ablative removal of corneal tissue to a uniform depth, wherein original anterior surface curvature is replicated at the floor of the constant-diameter incision, but the assumption is made in said patents that the laser-beam section is characterized by a homogeneous distribution of flux density. Subsequent experience is that a homogeneous distribution of flux density is not readily achievable with currently available excimer lasers, which characteristically have a more or less Gaussian profile in one meridian, and a nearly "top-hat" profile in the orthogonal meridian; and even if optical or beam-rotating means are employed in an effort to make the flux-density profile more nearly uniform, the result is generally an axisymmetric profile, with approximately parabolic fall-off, from axis to edge. Other laser types may deliver a beam with a Gaussian fall-off, from axis to edge, but for simplified present discussion, the expression "parabolic" is adopted in reference to an ablating-laser beam section, with fall-off from axis to edge.

Fig. 8 illustrates that, despite intensity fall-off in the laser beam, the presently described apparatus can be used to prepare a cornea to a uniform depth Z for reception of a corneal transplant of given radius 70. To this end, the microprocessor will have been programmed to select one of the apertures of masking disc 18, in conjunction with suitable adjustment of zoom lens 14, whereby the beam section impacting the cornea is of the given radius 70. But since the beam section has (or with beam rotation delivers) a fall-off intensity distribution that is somewhat parabolic, the initial anterior-surface profile 71 cannot be replicated (at 71') at the desired depth Z; on the other hand, exposure to the parabolic intensity distribution results in an ablated floor profile 72 which is of different curvature, ranging from the full desired depth Z on the axis of beam delivery, but falling off to a lesser depth Z₁ at the radius 70 of the preparation; the extent of the short fall at radius 70 is designated ΔZ in Fig. 8.

Fig. 8A illustrates that, with microprocessor control of laser-beam exposure through successive apertures of a hyperopia-correcting mask (such as the mask 17), and with radius 70 maintained as described above (e.g., by one of the apertures of disc 18, in conjunction with the indicated adjustment of zoom lens 14), it is possible to continue the program described in connection with Fig. 8, using what would otherwise be a hyperopia-correcting program. The result of such a program is an incrementally stepped abated-surface floor, shown with exaggeration by solid line 73; these steps will be understood to correspond with successive annuli (74d through 74h) of laser-beam exposure through mask apertures such as the apertures 41d through 41h of mask 17.

Fig. 8B illustrates further that, again with suitable microprocessor control, involving coordinated use of mask-indexing and zoom-lens variation, a much more smoothly developed floor profile 75 can be achieved, without involving zoom-lens magnification beyond a predetermined range limit. In such an application, a fixed outer limit of exposed area must account for the desired constant radius 70 of the ablation process, and this fixed outer limit must be determined by means (e.g., at shutter 21) between the zoom lens 14 and the patient's cornea; this is a simple matter of assuring that when shutter 21 (Fig. 1) is open, it presents a circular aperture which determines the constant radius 70 at beam impingement upon the cornea.

If it is determined that the range of zoom-lens magnification is 1:2, then an indexible mask of the nature shown in Fig. 2A need only have four apertures; but if the range of zoom-lens magnification is to be 1:1.414, then there should be twice as many apertures. In Fig. 8B, the symbolism R₁, R₂ ...R₈ will be understood to identify successive outer radii attributable to the central opaque spot in each of a succession of eight apertures in an indexible mask 17; and the additional parenthetical and primed entry adjacent each such radius is to be understood as designating the radius to which the coordinated zoom-lens adjustment can account for radius expansion for exposure via a particular indexed aperture. Thus, for example, for the mask aperture having the smallest-diameter central spot, the designation R₁ (R₂') is to be understood as indicating the range of radius expansion (i.e., R₁ to R₂) available for this smallest central spot, within the indicated 1:1.414 magnification range of a coordinated control of zoom-lens setting. Similar designations R₂ (R₃'),R₃ (R₄'), R₄ (R₅') ... R₈ (R₉') apply for the respective indexed apertures of the mask, as the central spot is indexed to grow, for each successive aperture use of the same controlled 1:1.414 magnification range of zoom lens 14. And it is to be understood that coordinated equalizing adjustment is preferred for beam intensity control as a function of zoom-lens magnification, as explained above in connection with Fig. 1A. The result is the smoothed profile 75, representing essentially constant depth of ablating penetration of the cornea, to depth Z.
Fig. 9 illustrates much of what has been described in connection with Figs. 8, 8A, 8B, except for the formation of a blend or bevel 76 beyond the optically used radius, to encourage epithelium regrowth over a newly sculpted surface 75' of constant depth Z with respect to the original contour 71' of the anterior surface of the cornea. But, in the case of Fig. 9, the original contour 71' of an otherwise perfect cornea, is marred by a scar or other wound 77 which has only partially invaded the cornea, and the object of the sculpturing procedure is to select a constant depth Z which is sufficient to eliminate the wound, so that the patient's cornea can be resurfaced to his own good curvature at 75', with relatively prompt epithelium regrowth over the refinished surface.

To prepare for the operation of Fig. 9, the volume of wound 77 should be filled with epithelium or other suitably viscous material (such as a drop of 1% methyl cellulose) having substantially the same ablation response to the laser beam as is the case for corneal tissue to be ablated to depth Z; wound filling should be made to essentially the original contour 71'.

Presently described apparatus can perform the involved operation, in the indicated circumstances of a laser beam having a parabolic fall-off distribution of flux density, under the following set-up conditions, for a two-step procedure:
STEP 1. The control of iris 16 is programmed to determine the outer-radius limit of beam exposure to the cornea in synchronized coordination with instantaneous zoom-lens setting, so as to determine the outer radius (70 + ΔR) of blend 76 at the outset, and so as to gradually reduce this outer radius by the decrement ΔR', as of the time T₁ when steady exposure (determined by the thus-varying iris aperture) has achieved the depth Z of abated penetration on the axis of beam delivery.
STEP 2. Having achieved STEP 1, shutter 21 is actuated to transiently cut off beam delivery while a suitable hyperopia-reducing masking disc 17 is indexed into its first operative position, e.g., with its central opaque spot, in coordination with zoom-lens control, determining varying radius in the range from R₁ to R₂ (as in Fig. 8B); for purposes of STEP 2, it will be understood that the annular apertures of disc 17 will have been selected such that the outer radius of the annular aperture is not a limitation on delivered outer radius (70 + ΔR - ΔR') of the laser beam, so that the STEP-1 program of iris coordination with zoom-lens control can continue the further development of the blend or bevel 76 throughout the full STEP-2 exposure, from time T₁ to time T₂. Shutter operation repeats for each indexing of successive apertures of the mask 17; and zoom-lens control in the range 1:1.414 together with beam-equalizing control, recycle as appropriate for exposures through each of the successively indexed apertures of mask 17. At time T₂, the operation is complete, and the desired contours 75' and 76 have been established. The patient need only await the approximately two-day period for epithelium regrowth, and he can then use his resurfaced cornea.

It will be understood that the described procedure in connection with Fig. 9 is not necessarily limited to an otherwise perfect cornea that has been scarred or otherwise wounded. It could be that the patient with the wounded cornea had an optical curvature deficiency which he had previously corrected by a spectacle or contact lens. In that case, and faced with the prospect of laser surgery to eliminate his wound, he could select at the same time to have his original curvature deficiency corrected by laser surgery, as part of the same procedure which eliminates the wound on his cornea. That the described apparatus is programmable for such purposes, is evident from the above-described examples, even in the presence of a parabolic or other characteristic fall-off of intensity distribution in the laser beam.

## Claims

1. Apparatus for performing ophthalmological surgery by selective ablation of the optically used region of the anterior surface of the cornea with penetration into the stroma to achieve a volumetric removal of cornea tissue providing for corneal curvature correction, said apparatus comprising laser means (13) for producing an output beam having an intensity which is limited per unit time to ablate but a fraction of a predetermined maximum ablation depth into the stroma, means (10) for directing said laser output beam onto the eye which is to be treated, and control means (14-19) for adjustably determining the laser beam cross-section at cornea impingement in said optically used region in a controlled manner in the course of a surgical treatment so that sculpturing action for curvature correction results from a controlled variation in the area of the cornea that is irradiated and a corresponding controlled variation in the cumulative ablative flux delivered to different areas of said optically used region of the cornea so as to obtain said corneal curvature correction, said apparatus being characterized in that said control means (14-19) comprises the combination in the laser beam path of at least one mask (17, 18, 19) having a plurality of mask apertures (41) of different areas that are selectively introducible into the laser beam path for correspondingly determining the laser beam cross-section at corneal impingement and a variable magnification zoom lens (14), and means (23) arranged to controllably adjust the setting of the zoom lens (14) in the course of corneal irradiation through each mask aperture (41) in a series of progressively different area mask apertures (41) in order to further subdivide the steps of progression of the laser beam cross-section at corneal impingement that are achieved by irradiation of the optically used region of the cornea through said series of different mask apertures (41) thereby to obtain a substantially smooth change in corneal curvature profile.

2. Apparatus according to claim 1 which further includes control means (23) for varying the intensity of the laser beam to compensate for flux-density variations resulting from variation of the setting of the zoom-lens.

3. Apparatus according to claim 2 wherein said control means (23) includes a microprocessor having an intensity-control connection to said laser means (13) and a magnification-control connection to said zoom-lens (14).

4. Apparatus according to claim 2 or 3 which further includes variable-density filter means (81, 82) interposed between said laser means (13) and said zoom-lens (14).

5. Apparatus according to claim 4, wherein said variable-density filter means (81, 82) comprises two like variable-density filters arranged in mutually opposite directions of varying density and coupled for opposing displacements through the laser beam in synchronism with variation of the zoom-lens setting.

6. Apparatus according to any of the preceding claims, in which a plurality of said masks (17, 18, 19) are provided in an axially separated array and are separately indexible to place different mask apertures in the beam path, each of said masks (17, 18, 19) having a differently characterized progression of different area apertures (41), and one of the apertures (40) of each mask being of such an area as to avoid limitation of the beam-section-limiting ability of any aperture of any other mask of said plurality.

7. Apparatus according to any of the preceding claims, in which said at least one mask includes an aperture of outer radius which when projected onto the cornea via said zoom-lens is greater than that of the maximum optically used area of the cornea that is to be ablated, said control means (14-19) further including adjustable iris means (16) and means (23) for co-ordinating the iris setting with the zoom-lens setting and the current mask aperture so as to effect a relatively smoothly bevelled annular zone of blending slope outwardly from said maximum optically used area.

8. Apparatus according to any of claims 1 to 6, in which the apertures of said at least one of said masks are circularly annular and of progressively changing inner radius, and have an outer radius which when projected onto the cornea via said zoom-lens is greater than that of the maximum optically used area of the cornea that is to be ablated, said control means (14-19) further including adjustable iris means (16) and means (23) for co-ordinating the iris setting with the zoom-lens setting and the current mask aperture so as to effect a relatively smoothly profiled hyperopia-correcting diopter change within said maximum curvature-correcting area and also to effect a relatively smoothly bevelled annular zone of blending slope outwardly from said area of diopter-changing curvature correction.

9. Apparatus according to any of the preceding claims, in which the mask apertures of said at least one mask (18) are circular and of progressively changing radius, whereby to effect a relatively smoothly profiled myopia-correcting diopter change.

10. Apparatus according to any of the preceding claims, in which the mask apertures of said at least one mask (19) are elongate rectangular and of progressively changing lateral width, and selectively operable means (46) are provided for orienting the elongation axes of said apertures in accordance with a predetermined orientation of desired astigmatism correction whereby to effect a relatively smoothly profiled cylindrical diopter change.

11. Apparatus according to any of the preceding claims, in which said control means further includes shutter means (21) operable to preclude laser-beam impingement at the cornea upon completion of beam exposure for each utilized mask aperture and for the period of movement into the operative position of the next mask aperture and the period of zoom-lens resetting for operative use of said next mask aperture.

12. Apparatus according to any of the preceding claims, in which the number of mask apertures and the relation of area change between one and the next mask aperture through the progression of area change is selected for limitation of requisite zoom-lens magnification to the range 1:2 or less, for each of the indexed positions of said mask, whereby the variation in energy density at the cornea is within the range 1:4.

13. Apparatus according to claim 12, in which the number of mask apertures and the relation of area change between one and the next mask aperture through the progression of area change is selected for limitation of requisite zoom-lens magnification to the range 1:1.414 or less, for each of the indexed positions of said mask, whereby the variation in energy density at the cornea is within the range 1:2.

## Patentansprüche

1. Vorrichtung zur Durchführung einer ophthalmologischen Operation durch selektive Ablatio des optisch genutzten Bereichs der Vorderfläche der Hornhaut mit Eindrigen in das Stroma, um eine volumetrische Entfernung von Hornhautgewebe, die für eine Hornhautkrümmungskorrektur sorgt, zu erreichen, wobei die genannte Vorrichtung eine Lasereinrichtung (13) zur Erzeugung eines Ausgangsstrahls mit einer Intensität, die pro Zeiteinheit begrenzt ist, um nur einen sehr geringen Teil einer vorbestimmten maximalen Abtragtiefe in das Stroma hinein wegzunehmen, Mittel (10) zum Richten des besagten Laserausgangsstrahls auf das zu behandelnde Auge und Regeleinrichtungen (14 - 19) zur justierbaren Bestimmung des Laserstrahlquerschnitts am kornealen Einfall in den erwähnten optisch genutzten Bereich in einer kontrollierten Weise im Verlauf der operativen Behandlung, so daß eine Formgebungswirkung für eine Krümmungskorrektur aus einer geregelten Veränderung in der Fläche der Hornhaut, die bestrahlt wird, und einer entsprechenden geregelten Veränderung im kumulativen abtragenden, den verschiedenen Flächen des erwähnten optisch genutzten Bereichs der Hornhaut zugeführten Fluß resultiert, um die besagte Hornhautkrümmungskorrektur zu erlangen, umfaßt,
dadurch gekennzeichnet, daß die erwähnten Regeleinrichtungen (14 - 19) die Kombination im Laserstrahlengang von mindestens einer Maske (17, 18, 19) mit einer Mehrzahl von Maskenaperturen (41) mit unterschiedlichen Flächeninhalten, die selektiv in den Laserstrahlengang einführbar sind, um entsprechend den Laserstrahlquerschnitt am kornealen Einfall festzusetzen, und eines Zoom-Objektivs (14) mit veränderlicher Vergrößerung sowie eine Einrichtung (23), die ausgestaltet ist, um kontrollierbar die Einstellung des Zoom-Objektivs (14) im Verlauf der Hornhautbestrahlung durch jede Maskenapertur (41) in einer Folge von Maskenaperturen (41) mit progressiv unterschiedlichen Flächeninhalten zu justieren, um die Progressionsstufen des Laserstrahlquerschnitts am kornealen Einfall, die durch Bestrahlung des optisch genutzten Bereichs der Hornhaut durch die genannte Folge von verschiedenen Maskenaperturen (41) hindurch erlangt werden, weiter zu unterteilen und dadurch eine im wesentlichen stetige Änderung im Hornhautkrümmungsprofil zu erhalten, umfassen.

2. Vorrichtung nach Anspruch 1, die ferner Regeleinrichtungen (23) zur Veränderung der Intensität des Laserstrahls enthält, um aus einer Änderung der Einstellung des Zoom-Objektivs resultierende Flußdichteänderungen zu kompensieren.

3. Vorrichtung nach Anspruch 2, in welcher die genannten Regeleinrichtungen (23) einen Mikroprozessor einschließen, der eine Intensitätsregelverbindung zur genannten Lasereinrichtung (13) und eine Vergrößerungsregelverbindung zum genannten Zoom-Objektiv (14) besitzt.

4. Vorrichtung nach Anspruch 2 oder 3, die ferner eine zwischen die genannte Lasereinrichtung (13) und das genannte Zoom-Objektiv (14) eingesetzte Verlauffiltereinrichtung. (81, 82) enthält.

5. Vorrichtung nach Anspruch 4, in welcher die genannte Verlauffiltereinrichtung (81, 82) zwei gleiche Verlauffilter umfaßt, die in wechselseitig entgegengesetzten Richtungen sich ändernder Dichte angeordnet und für entgegengesetzte Verlagerungen durch den Laserstrahl hindurch synchron mit einer Änderung in der Zoom-Objektiveinstellung gekoppelt sind.

6. Vorrichtung nach einem der vorhergehenden Ansprüche, in welcher eine Mehrzahl der genannten Masken (17, 18, 19) in einer axial separierten Reihe vorgesehen und einzeln schaltbar sind, um unterschiedliche Maskenaperturen in den Strahlengang einzusetzen, wobei jede der genannten Masken (17, 18, 19) eine unterschiedlich charakterisierte Progression von Aperturen (41) unterschiedlicher Flächeninhalte hat und eine der Aperturen (40) einer jeden Maske von solchem Flächeninhalt ist, um eine Einschränkung des Strahlquerschnitt-Begrenzungsvermögens irgendeiner Apertur von irgendeiner anderen Maske aus der besagten Mehrzahl zu vermeiden.

7. Vorrichtung nach einem der vorhergehenden Ansprüche, in welcher die erwähnte mindestens eine Maske eine Apertur mit einem Außenradius enthält, der, wenn er auf die Hornhaut mittels des genannten Zoom-Objektivs projiziert wird, größer ist als derjenige des maximalen optisch genutzten Bereichs der Hornhaut, der abzutragen ist, wobei die besagten Regeleinrichtungen (14 - 19) ferner ein einstellbares Aperturblendenorgan (16) und Einrichtungen (23) zur Koordination der Aperturblendeneinstellung mit der Zoom-Objektiveinstellung und der gegenwärtigen Maskenapertur einschließen, um eine relativ glatt abgeschrägte Ringzone einer Übergangsschräge außenseitig des erwähnten maximalen optisch genutzten Bereichs zu bewerkstelligen.

8. Vorrichtung nach einem der Ansprüche 1 bis 6, in welcher die Aperturen der erwähnten mindestens einen der Masken kreisringförmig sowie von progressiv sich änderndem Innenradius sind und einen Außenradius haben, der bei seiner Projektion auf die Hornhaut mittels des genannten Zoom-Objektivs größer ist als derjenige des maximalen optisch genutzten Bereichs der Hornhaut, der abzutragen ist, wobei die besagten Regeleinrichtungen (14 - 19) ferner ein einstellbares Aperturblendenorgan (16) und Einrichtungen (23) zur Koordination der Aperturblendeneinstellung mit der Zoom-Objektiveinstellung und der gegenwärtigen Maskenapertur einschließen, um eine relativ glatt profilierte, eine Hyperopie korrigierende Dioptrieänderung innerhalb des erwähnten maximalen krümmungskorrigierenden Bereichs zu bewirken und auch eine relativ glatt abgeschrägte Ringzone einer Übergangsschräge außenseitig des erwähnten Bereichs einer dioptrieändernden Krümmungskorrektur zu bewerkstelligen.

9. Vorrichtung nach einem der vorhergehenden Ansprüche, in welcher die Maskenaperturen der erwähnten wenigstens einen Maske (18) kreisförmig und von progressiv sich änderndem Radius sind, um dadurch eine relativ glatt profilierte, eine Myopie korrigierende Dioptrieänderung zu bewirken.

10. Vorrichtung nach einem der vorhergehenden Ansprüche, in welcher die Maskenaperturen der erwähnten wenigstens einen Maske (19) länglich rechteckig sowie von sich progressiv ändernder Querbreite sind und selektiv betätigbare Mittel (46) für ein Ausrichten der Längsachsen der genannten Aperturen in Übereinstimmung mit einer vorbestimmten Orientierung einer gewünschten Astigmatismuskorrektur vorgesehen sind, um dadurch eine relativ glatt profilierte zylindrische Dioptrieänderung zu bewirken.

11. Vorrichtung nach einem der vorhergehenden Ansprüche, in welcher die erwähnten Regeleinrichtungen ferner ein Verschlußorgan (21) enthalten, das betätigbar ist, um einen Laserstrahleinfall auf die Hornhaut bei Beendigung einer Strahlbelichtung für jede verwendete Maskenapertur sowie für die Bewegungszeitspanne in die operative Position der nächsten Maskenapertur und für die Zeitspanne einer Zoom-Objektivrückstellung zur operativen Verwendung der genannten nächsten Maskenapertur auszuschließen.

12. Vorrichtung nach einem der vorhergehenden Ansprüche, in welcher die Anzahl der Maskenaperturen und die Beziehung einer Flächeninhaltänderung zwischen der einen sowie der nächsten Maskenapertur durch die Progression der Flächeninhaltänderung für eine Begrenzung einer erforderlichen Zoom-Objektivvergrößerung auf den Bereich 1:2 oder weniger für jede der weitergeschalteten Stellungen der genannten Maske ausgewählt wird, wodurch die Änderung in der Energiedichte an der Hornhaut innerhalb des Bereichs 1:4 liegt.

13. Vorrichtung nach Anspruch 12, in welcher die Anzahl der Maskenaperturen und die Beziehung einer Flächeninhaltänderung zwischen der einen sowie der nächsten Maskenapertur durch die Progression der Flächeninhaltänderung für eine Begrenzung einer erforderlichen Zoom-Objektivvergrößerung auf den Bereich 1:1,414 oder weniger für jede der weitergeschalteten Positionen der genannten Maske ausgewählt wird, wodurch die Änderung in der Energiedichte an der Hornhaut innerhalb des Bereichs 1:2 liegt.

## Revendications

1. Appareil pour réaliser de la chirurgie ophtalmologique par ablation sélective de la région optiquement utilisée de la surface antérieure de la cornée avec pénétration dans le stroma, pour réaliser un retrait volumétrique de tissu cornéen, assurant une correction de courbure cornéenne, ledit appareil comprenant :
- un moyen laser (13) pour produire un faisceau de sortie ayant une intensité qui est limitée par unité de temps pour n'enlever qu'une fraction d'une profondeur d'ablation maximale prédéterminée dans le stroma ;
- un moyen (10) pour diriger ledit faisceau de sortie du laser sur l'oeil qui doit être traité ; et
- un moyen de commande (14 - 19) pour déterminer de façon ajustable la section transversale du faisceau laser au niveau de l'impact sur la cornée dans ladite région optiquement utilisée d'une manière contrôlée au cours d'un traitement chirurgical, de telle sorte que l'action de sculpture pour la correction de courbure résulte d'une variation contrôlée dans la zone de la cornée qui est irradiée et d'une variation contrôlée correspondante dans le flux d'ablation cumulé adressé aux différentes zones de ladite région optiquement utilisée de la cornée, de façon à obtenir ladite correction de courbure cornéenne,
ledit appareil étant caractérisé par le fait que ledit moyen de commande (14 - 19) comprend la combinaison dans la trajectoire du faisceau laser d'au moins un masque (17, 18, 19) ayant une pluralité d'ouvertures de masque (41) de différentes surfaces qui peuvent être introduites sélectivement dans la trajectoire du faisceau laser pour déterminer de façon correspondante la section transversale du faisceau laser au niveau de l'impact sur la cornée et d'une lentille à focale variable à grossissement variable (14), et un moyen (23) disposé pour ajuster de façon contrôlable le positionnement de la lentille (14) à focale variable au cours de l'irradiation de la cornée à travers chaque ouverture de masque (41) d'une série d'ouvertures de masque (41) de surfaces progressivement différentes, afin de subdiviser encore les étages de progression de la section transversale de faisceau laser au niveau de l'impact sur la cornée qui sont obtenus par irradiation de la région optiquement utilisée de la cornée à travers ladite série de différentes ouvertures de masque (41), pour obtenir ainsi un changement sensiblement sans inégalités dans le profil de courbure cornéenne.

2. Appareil selon la revendication 1, qui comprend en outre un moyen de commande (23) pour faire varier l'intensité du faisceau laser afin de compenser les variations de densité de flux résultant de la variation du positionnement de la lentille à focale variable.

3. Appareil selon la revendication 2, dans lequel ledit moyen de commande (23) comprend un microprocesseur ayant une connexion de commande d'intensité audit moyen laser (13) et une connexion de commande de grossissement à ladite lentille (14) à focale variable.

4. Appareil selon la revendication 2 ou 3, qui comprend en outre un moyen de filtre à densité variable (81, 82) interposé entre ledit moyen laser (13) et ladite lentille (14) à focale variable.

5. Appareil selon la revendication 4, dans lequel ledit moyen de filtre à densité variable (81, 82) comprend deux filtres de densité variable similaires, disposés suivant des directions mutuellement opposées de densité variable et couplés en vue de déplacements opposés à travers le faisceau laser en synchronisme avec la variation du positionnement de la lentille à focale variable.

6. Appareil selon l'une des revendications précédentes, dans lequel plusieurs desdits masques (17, 18, 19) sont disposés dans un alignement axialement séparé et sont indexables de façon séparée pour placer différentes ouvertures de masques dans la trajectoire du faisceau, chacun desdits masques (17, 18, 19) ayant une progression caractérisée de façon différente des ouvertures de différentes surfaces (41), et l'une des ouvertures (40) de chaque masque étant d'une surface telle qu'elle empêche la limitation de l'aptitude à limiter la section de faisceau de n'importe quelle ouverture de n'importe quel autre masque de ladite pluralité de masques.

7. Appareil selon l'une des revendications précédentes, dans lequel ledit au moins un masque comprend une ouverture de rayon externe qui, lorsqu'elle est projetée sur la cornée par l'intermédiaire de ladite lentille à focale variable, est plus grande que celle de la zone optiquement utilisée maximale de la cornée qui doit être enlevée, ledit moyen de commande (14 - 19) comprenant en outre un moyen d'iris ajustable (16) et un moyen (23) pour coordonner le positionnement de l'iris avec le positionnement de la lentille à focale variable et l'ouverture de masque courante afin d'effectuer une zone annulaire inclinée de façon relativement douce de rattrapage de la cornée non enlevée vers l'extérieur à partir de ladite zone optiquement utilisée maximale.

8. Appareil selon l'une des revendications 1 à 6, dans lequel les ouvertures dudit au moins l'un desdits masques sont circulairement annulaires et de rayon interne changeant progressivement, et ont un rayon externe qui, lorsqu'il est projeté sur la cornée par l'intermédiaire de ladite lentille à focale variable, est plus grand que celui de la zone optiquement utilisée maximale de la cornée qui doit être enlevée, ledit moyen de commande (14 - 19) comprenant en outre un moyen d'iris ajustable (16) et un moyen (23) pour coordonner le positionnement de l'iris avec le positionnement de la lentille à focale variable et l'ouverture de masque courante, afin d'effectuer un changement de dioptrie de correction d'hypermétropie à profil relativement sans inégalités à l'intérieur de ladite zone de correction de courbure maximale et également afin d'effectuer une zone annulaire inclinée de façon relativement douce de rattrapage de la cornée non enlevée vers l'extérieur à partir de ladite zone de correction de courbure de changement de dioptrie.

9. Appareil selon l'une des revendications précédentes, dans lequel les ouvertures de masque dudit au moins un masque (18) sont circulaires et de rayon changeant progressivement, pour effectuer ainsi un changement de dioptrie de correction de myopie à profil relativement sans inégalités.

10. Appareil selon l'une des revendications précédentes, dans lequel les ouvertures de masque dudit au moins un masque (19) sont rectangulaires allongées, et de largeur latérale changeant progressivement, et des moyens actionnables sélectivement (46) sont prévus pour orienter les axes d'allongement desdites ouvertures conformément à une orientation prédéterminée de correction d'astigmatisme souhaitée, pour effectuer ainsi un changement de dioptrie cylindrique à profil relativement sans inégalités.

11. Appareil selon l'une des revendications précédentes, dans lequel ledit moyen de commande comprend en outre un moyen d'obturateur (21), actionnable pour empêcher l'impact du faisceau laser au niveau de la cornée lors de l'accomplissement de l'exposition au faisceau pour chaque ouverture de masque utilisée et pendant la période de déplacement dans la position active de l'ouverture de masque suivante et la période de repositionnement de la lentille à focale variable pour une utilisation active de ladite ouverture de masque suivante.

12. Appareil selon l'une des revendications précédentes, dans lequel le nombre d'ouvertures de masque et la relation de changement de surface entre une ouverture de masque et la suivante grâce à la progression de changement de surface est choisi en vue d'une limitation du grossissement de la lentille à focale variable requis à la plage de 1:2 ou au-dessous, pour chacune des positions indexées dudit masque, ce par quoi la variation de la densité d'énergie au niveau de la cornée se situe à l'intérieur de la plage 1:4.

13. Appareil selon la revendication 12, dans lequel le nombre d'ouvertures de masque et la relation de changement de surface entre une ouverture de masque et la suivante grâce à la progression de changement de surface sont choisis en vue d'une limitation de grossissement de la lentille à focale variable requis à la plage de 1:1,414 ou au-dessous, pour chacune des positions indexées dudit masque, ce par quoi la variation de la densité d'énergie au niveau de la cornée se situe à l'intérieur de la plage 1:2.
